(19) Europäisches Patentamt / European Patent Office / Office européen des brevets

(11) **EP 3 977 124 B1**

(12) **EUROPEAN PATENT SPECIFICATION**

(45) Date of publication and mention of the grant of the patent:
**06.03.2024 Bulletin 2024/10**

(21) Application number: **20742874.9**

(22) Date of filing: **22.05.2020**

(51) International Patent Classification (IPC):
**G01N 33/543** (2006.01)   **G01N 33/84** (2006.01)

(52) Cooperative Patent Classification (CPC):
**G01N 33/5432; G01N 33/84**

(86) International application number:
**PCT/IB2020/054887**

(87) International publication number:
**WO 2020/240377 (03.12.2020 Gazette 2020/49)**

(54) **LIPOSOME COMPOSITION AND DOSING METHOD BASED ON USE OF THE SAME**

LIPOSOMZUSAMMENSETZUNG UND AUF DEREN VERWENDUNG BASIERENDES DOSIERVERFAHREN

COMPOSITION DE LIPOSOMES ET PROCÉDÉ DE DOSAGE BASÉ SUR SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2019 IT 201900007222**

(43) Date of publication of application:
**06.04.2022 Bulletin 2022/14**

(73) Proprietor: **Universita' Degli Studi di Torino**
**10124 Torino (IT)**

(72) Inventors:
• **AIME, Silvio**
**10124 Torino (IT)**
• **DELLI CASTELLI, Daniela**
**10124 Torino (IT)**
• **FERRAUTO, Giuseppe**
**10124 Torino (IT)**
• **TRIPEPI, Martina**
**10124 Torino (IT)**

(74) Representative: **Studio Torta S.p.A. et al**
**Via Viotti, 9**
**10121 Torino (IT)**

(56) References cited:
**WO-A1-2013/011055    WO-A1-2015/060819**
**US-A- 4 962 022**

• **HORIE ET AL: "Fluorometric immunoassay based on pH-sensitive dye-encapsulating liposomes and gramicidin channels", ANALYTICAL BIOCHEMISTRY, ELSEVIER, AMSTERDAM, NL, vol. 369, no. 2, 5 September 2007 (2007-09-05), pages 192-201, XP022231374, ISSN: 0003-2697, DOI: 10.1016/J.AB.2007.07.007 cited in the application**

**Description**

CROSS-REFERENCE TO RELATED APPLICATIONS

This patent application claims priority from Italian patent application no. 102019000007222 filed on May 24, 2019, the entire disclosure of which is incorporated herein by reference.

TECHNICAL FIELD

The present invention concerns the use of a composition liposomes in a dosing method, preferably an *in vitro* diagnostic method, for example an immunometric method.

PRIOR ART

Over the last 50 years numerous and increasingly refined methods have been developed for detecting diagnostic markers in biological fluids *in vitro*. These analyses are based mainly on antigen/antibody detection (immunoassay). Of these methods, those in which the target molecule adheres or binds specifically to a solid support appear to be more efficient. Immunoenzymatic analyses for the detection of analytes of diagnostic interest have been the subject of various studies and patents.[1,2] One of the most widely used methods is enzyme-linked immunosorbent assay (ELISA) which consists in detecting chromatic variations induced by the specific recognition between antigen and antibody. There are four types of ELISA test: i) direct, ii) indirect, iii) sandwich and iv) competitive. All these methods require the use of a spectrophotometer to quantify the antigen (pM sensitivity).[3] Since many biomarkers relevant in diagnostic terms are present in biological fluids at low concentration, there is the need to develop innovative diagnostic assays having greater sensitivity. Furthermore, analysis costs need to be reduced as far as possible.

Some variations of the ELISA method reported in the literature are magnetic and radio immunoenzymatic assays, which have greater sensitivity but require the use of more costly instrumentation than a spectrophotometer and highly qualified personnel.[4,5]

A method that has been widely investigated over the last decades to increase *in vitro* assay sensitivity is the use of nanosystems including liposomes, characterized by a large aqueous cavity easily adapted to containing substantial quantities of small molecules.[6] A strategy for carrying out immunological tests based on liposomes consists in replacing the enzyme able to generate the spectrophotometric variation with a marker molecule appropriately encapsulated in the liposomes. The marker molecule can be active in colorimetric,[7] fluorimetric,[8] chemiluminometric[9] or electrochemical[10] terms, thus providing different types of liposome-based immunological assay. The choice often depends on the detection instruments available in the laboratory.

There are many strategies for modifying an antigen or an antibody for immunological assays or introducing it into a liposomal membrane.[11-15] These easily reproducible procedures can be applied to a vast range of antigens and antibodies.[16]

Liang et al[20] have developed a method for detecting and dosing neuron-specific enolase by the use of glucose oxidase-loaded liposomes. In particular, once the liposome binds the neuron-specific enolase by the use of selective antibodies, glucose is added to the analysis solution. The addition of a surfactant causes rupture of the liposome and release of the glucose oxidase. The oxidation of the glucose by its enzyme generates a variation in the pH of the solution which is detected and quantified by means of a pH meter. The main drawback of this method is that it requires a further process phase in the analysis protocol with the relative criticalities and variabilities (addition of the substrate (glucose) to the analysis solution). Furthermore, the costs of preparation of a liposome containing an enzyme are high. Any storage of an enzyme requires particular attention.

US4962022 discloses liposome compositions comprising liposomes in an aqueous buffer at a pH of 6.5-8.5.

Horie et al. (2007), Analytical Biochemistry, vol. 369, p. 192-201, doi:10.1016/j.ab.2007.07.007, discloses fluorometric immunoassays based on pH-sensitive dye-encapsulating liposomes.

The need is felt in the art for new liposome compositions without the drawbacks of the known systems.

OBJECT OF THE INVENTION

The object of the present invention is therefore to provide liposomes that are easy and inexpensive to create, which allow the detection and dosing of an analyte with good sensitivity and using simple instruments present in any chemical laboratory.

Said object is achieved by means of an analyte dosing method according to claim 1.

In particular, a liposome composition is provided containing an aqueous solution having a first pH and presenting on the surface at least one specific ligand for an analyte of interest contained in a solution having a second pH, said first pH being different from said second pH, for use in a dosing method (not claimed) .

The aqueous solution contained in the liposomes can consist of an aqueous solution of a base, an aqueous solution of an acid or a buffer solution.

Preferably, the aqueous solution of a base is an aqueous solution in which the base is chosen from the group consisting of sodium hydroxide, lithium hydroxide, potassium hydroxide and ammonia.

Preferably, the solution of an acid is an aqueous solution in which the acid is chosen from the group consisting of hydrochloric acid, sulphuric acid, nitric acid, hydroiodic acid, perchloric acid, hydrobromic acid, phosphoric acid and acetic acid.

Preferably, the buffer solution is a solution chosen from the group consisting of bicarbonate buffer, phosphate buffer, acetate buffer, carbonate buffer and citrate buffer.

Advantageously, the liposomes according to the invention do not contain any active molecule such as, for example, enzymes, and do not require the use of further components. They are therefore very simple systems which allow accurate sensitive detection of the analyte, without requiring the use of sophisticated instrumentation.

The liposomes of the invention can be used in a quali- and quantitative dosing method, also for diagnostic purposes.

In particular, the liposomes of the invention can be used in immunoassays and tests *in vitro* for determining specific analytes useful as markers for early diagnosis and monitoring of pathological states such as tumours, cardiovascular diseases, infectious diseases and metabolic diseases. Said markers can be present in low concentration in biological fluids such as whole blood, plasma, serum, nasal secretions, lymph, expectorate, saliva, urine, sperm, vaginal fluid, sweat, transdermal exudate, synovial liquid, cerebrospinal liquid, amniotic liquid, vitreous humour, bile, gastric juice, colostrum and in all other biological fluids.

Preferably the solution containing the analyte is a solution with neutral pH.

The liposomes of the invention can be selected from the group consisting of:

- small unilamellar vesicles (SUV) having a hydrodynamic radius ranging between 40 nm and 100 nm;
- large unilamellar vesicles (LUV) having a hydrodynamic radius ranging between 100 nm and 500 nm;
- giant unilamellar vesicles (GUV) having a hydrodynamic radius ranging between 500 nm and 20 $\mu$m.

Furthermore, the liposomes of the invention can have lipid or polymer nature (polymersomes or dendrimerosomes).

A method for dosing an analyte of interest in a solution is also provided comprising the steps of:

a) making available a solution of said analyte of interest bound to a solid support, said solution having a first pH;
b) placing in contact with said analyte solution a liposome composition containing an aqueous solution having a second pH different from said first pH and presenting on the surface at least one ligand specific for said analyte of interest, so as to create a bond between said analyte and said ligand;
c) applying an external stimulus in order to cause release of the aqueous solution contained in said liposomes into the solution containing said analyte so as to obtain a third pH;
d) measuring said third pH;
e) calculating the dosage of said analyte.

The analyte and the ligand can be selected from the group consisting of antigen/antibody, immunoglobulin/protein A, biotin/avidin, hormone/receptor, complementary sequences of nucleic acids, aptamer/target molecule. However, almost any analyte/ligand pair can be used in the present method.

The bond between the analyte and the ligand can be a direct or indirect bond, namely mediated through further ligands. For example, the liposomes of the invention can be used in direct, indirect, sandwich or competitive ELISA.

Advantageously the method of the invention also lends itself to use in domestic diagnostic tests using indicators for display of the pH variations also with the naked eye.

The liposomes can be prepared with the thin film hydration method in the presence of the acid or basic or buffer solution (300 mOsm/L). The purification from non-internalized $OH^-/H_3O^+$ is carried out by means of:

i) neutralization (in the case of strong acid or base solutions, an equivalent of HCl or NaOH respectively is added), or
ii) dialysis or ultrafiltration (in the case of buffer solutions).

The suspension of the purified liposomes is therefore neutral in the presence of intact liposomes. The liposomes are induced to release their content by means of an external stimulus, preferably selected from the group consisting of ultrasounds, heat, presence of surfactants, osmotic stress and freeze-thaw cycles.

The release of the liposomal content induces a variation in the measurable pH value by the use of a pH meter or displayable through the colour change induced by a colorimetric/fluorescent pH indicator or, more in general, detectable by any pH-sensitive test.

BRIEF DESCRIPTION OF THE DRAWINGS

The present invention will now be described in detail with reference to the figures of the attached drawings, which show purely illustrative and non-limiting embodiment examples, in which:

- Figure 1 illustrates the calibration curve which correlates the quantity of liposomes of the invention that release OH⁻ and the pH variation.
- Figure 2 illustrates the calibration curve which correlates the quantity of liposomes of the invention that release OH⁻ and the pH variation for liposomes of the invention with mean diameter of 1 $\mu$m and containing 100 mM of NaOH (GUVs) and liposomes of the invention with mean diameter of 100 nm and containing 100 mM of NaOH (LUVs);
- Figure 3 illustrates the calibration curve which correlates the quantity of liposomes of the invention that contain a buffer solution of bicarbonate and the pH variation;
- Figure 4 illustrates the calibration curve which correlates the quantity of liposomes of the invention that contain a buffer solution of bicarbonate and the pH variation for liposomes of the invention with mean diameter of 1 $\mu$m and containing 150 mM of NaOH (circles) and compared with liposomes having a mean diameter of 100 nm and containing 150 mM of NaHCOs (squares);
- Figure 5 illustrates a first embodiment of the method of the invention;
- Figure 6 illustrates a second embodiment of the method of the invention;
- Figure 7 illustrates a third embodiment of the method of the invention;
- Figure 8 illustrates the pH values measured for the liposome suspension of example 1A;
- Figure 9 illustrates the pH values measured for the liposome suspension of example 1B;
- Figure 10 illustrates the pH values measured for the liposome's suspension of example 1C;
- Figure 11 illustrates the pH values measured in example 3A.

PREFERRED EMBODIMENT OF THE INVENTION

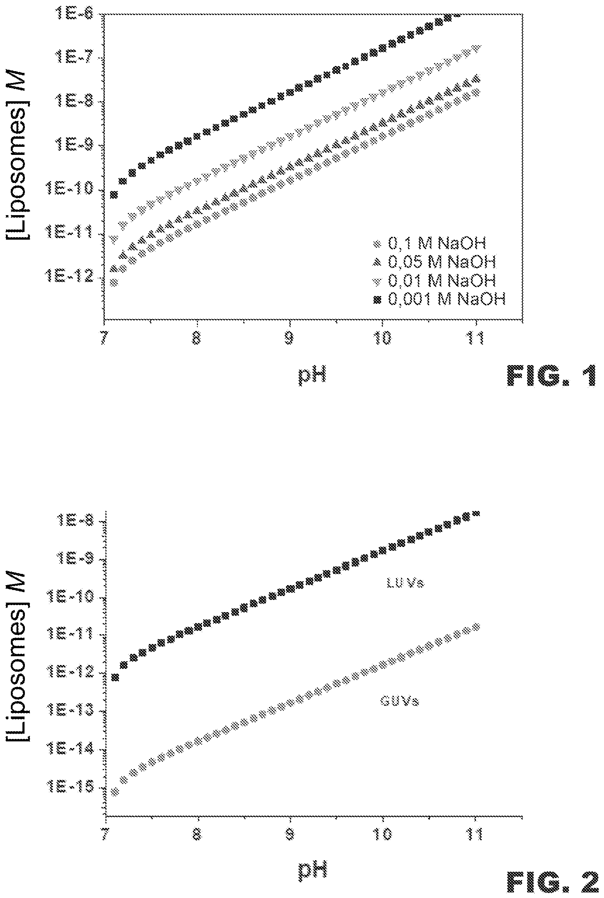

Figure 1 shows the correlation between the quantity of liposomes of the invention that release their content and the consequent increase in pH in a neutral solution calculated for a preparation of liposomes with a mean diameter of 150 nm and containing 100 mM or 50 mM or 10 mM or 1 mM of NaOH.

The curves were obtained considering that the number of liposomes in 1 L of solution that release their content is correlated with the concentration of OH⁻ according to the following relation (1):

$$[OH^-]_{released} = \text{No. liposomes (mol of strong base)}_{in\ single\ liposome} =$$
$$= \text{No. liposomes [strong base]}_{in\ single\ liposome}\ _{internal}\ V\ _{of\ liposome}$$

$$(1)$$

The number of liposomes can be converted into moles by simply dividing by the Avogadro constant (NA) and since the preceding relation (1) refers to 1 L of solution, the value in moles corresponds to the molar concentration value (relation 2).

$$(N° \text{ liposomes}/NA)/1\ L = [\text{ liposomes}]$$

$$(2)$$

In the present method, the pH is measured before and after inducing release of the liposome content. The pH of the analyte solution is neutral as long as the basic compound is trapped inside the liposomes.

Since:

$$[H_3O^+] = \frac{K_w}{[OH^-]_{equilibrium}}$$

where $K_w$ is the equilibrium constant of the water and $[OH^-]_{equilibrium}$ is the concentration of $OH^-$ at equilibrium, once the pH is known, it is possible to calculate $[OH^-]_{equilibrium}$ and from this value, by means of the following equation (3), it is possible to obtain $[OH^-]_{released}$:

$$[OH^-]_{released} = \frac{([OH^-]_{equilibrium})^2 - K_w}{[OH^-]_{equilibrium}}$$

$$(3)$$

By substituting equation 3 in equation 1 it is possible to obtain the calibration reported in Figure 1.

The sensitivity can be in the order of pM and is influenced by the concentration of $OH^-/H_3O^+$ trapped.

The possibility of drastically increasing the quantity of $OH^-/H_3O^+$ consists in the use of larger vesicles like the giant unilamellar vesicles (GUVs) (Figure 2).

The sensitivity that can be achieved using the giant liposomes (GUVs) is in the order of magnitude of fM, suitable for the dosage of less concentrated biomolecules.

Analogously, the same type of calibration and sensitivity can be shown in the case of liposomes containing an aqueous solution of HCl instead of NaOH.

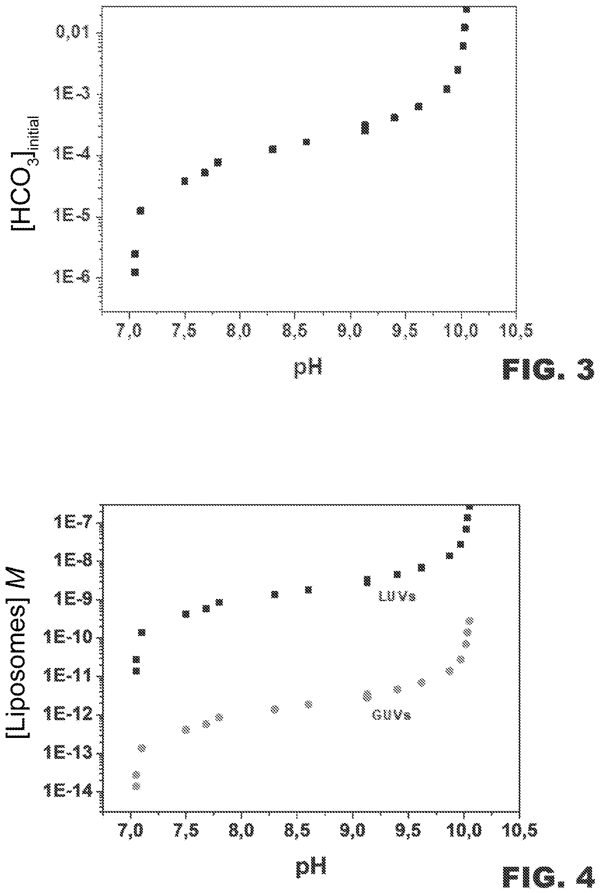

Figure 3 shows the calibration using a bicarbonate buffer and was obtained as follows:

i) 150 mM of sodium bicarbonate are dissolved in water;
ii) the solution is basified with NaOH up to pH 10.0; and subsequently
iii) the solution is diluted with bidistilled water at pH 7.0 and for each solution the pH is measured.

The curve shows a sensitivity region between 1 mM and 10 $\mu$M of initial NaHCOs in solution.

Once the diameter of the vesicles and concentration of NaHCOs are known, analogously to what was done previously, it is possible to correlate the number of liposomes that release their content with NaHCOs concentration by means of the following relation (4):

$$[liposomes] = N° \; liposomes \; per \; litre/NA =$$
$$= ((mol \; buffer)_{released} \, / (mol \; buffer)_{single \; liposome})/NA =$$
$$= (mol \; buffer)_{released}/([buffer]_{single \; liposome} * \; V_{intralipo}))/NA$$

$$(4)$$

Figure 4 shows the corresponding calibration curve.

The sensitivity for the GUV liposomes is in the fM range whereas for the LUVs it is in the pM range.

Many other different buffer solutions can be used as hydration solutions for this method.

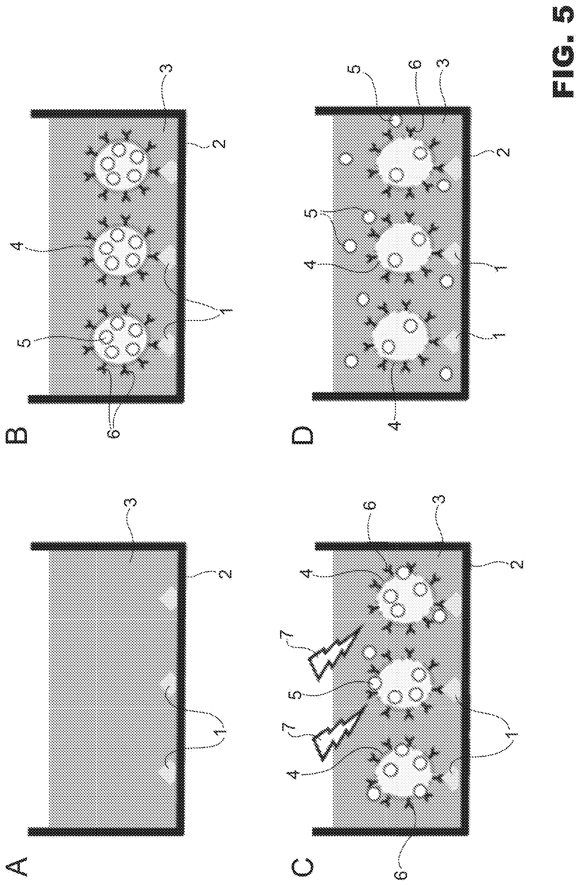

The liposomes of the invention can be used in direct, indirect or sandwich ELISA dosage methods which are schematized in figures 5-7.

In particular, with reference to figure 5, an analyte 1 is provided, for example an antigen, adhered to a solid support 2 in a solution 3.

A suspension of the liposomes 4 containing a solution 5 having a first pH, for example an acid pH of 5, and presenting on the surface a ligand 6, is added to said solution (figure 5B).

After an appropriate incubation time, which depends on the kinetics of the bond of the analyte/ligand pair, the non-bound liposomes are removed by means of washing cycles, for example with a solution of NaCl 0.15 M at pH 7.0. At this point a first measurement of the pH in the solution 3 is carried out.

The release of the aqueous solution 5 is induced by an external stimulus 7, in this case for example heat (Figure 5C).

Lastly, after a period of time appropriate for complete release of the aqueous solution 5 contained in the liposomes into the solution 3, a second pH measurement is taken which is then compared with the pH before release of the solution 5.

Measuring the pH of the solution after rupture of the liposomes, the quantity of liposomes bound to the ligand is easily quantifiable using the calibrations illustrated above.

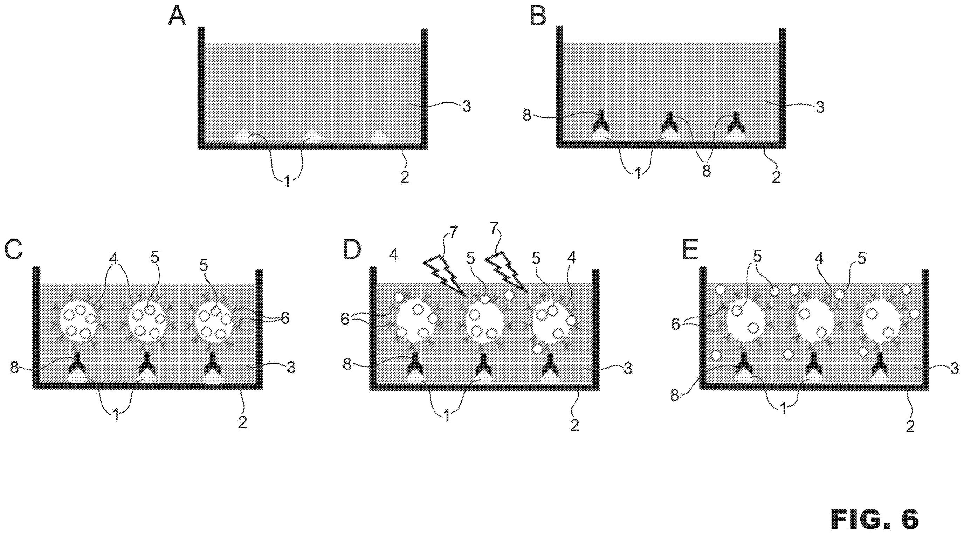

Figure 6 illustrates an alternative embodiment of the method of the invention. The details similar or equal to those already described in figure 5 are indicated for the sake of simplicity by the same reference numbers. In particular, a solution of a free secondary ligand 8 (e.g. primary antibody) (Fig. 6B) is added to the solution 3 of the analyte 1 on solid support 2 (Fig. 6A).

After an appropriate incubation time, which depends on the kinetics of the bond of the analyte/secondary ligand pair, the free secondary ligands 8 not bound are removed by means of washing cycles, for example with a solution of NaCl 0.15 M at pH 7.0.

A suspension of liposomes 4 is then added, adequately functionalized with a primary ligand 6 (e.g. secondary antibody) and containing an aqueous solution 5 having a pH different from that of the solution 3 to bind the secondary ligand (Fig. 6C). At this point, before the rupture of the liposomes, a first pH measurement is taken.

The release of the aqueous solution 5 is induced by an external stimulus 7, in this case for example heat (Figure 6D).

Lastly, after a time adequate for complete release of the aqueous solution 5 contained in the liposomes into the solution 3 (Fig. 6E), a second pH measurement is taken which is then compared with the pH before release of the solution 5.

By measuring the pH of the solution after rupture of the liposomes, the quantity of liposomes bound to the ligand is easily quantifiable using the calibrations illustrated above.

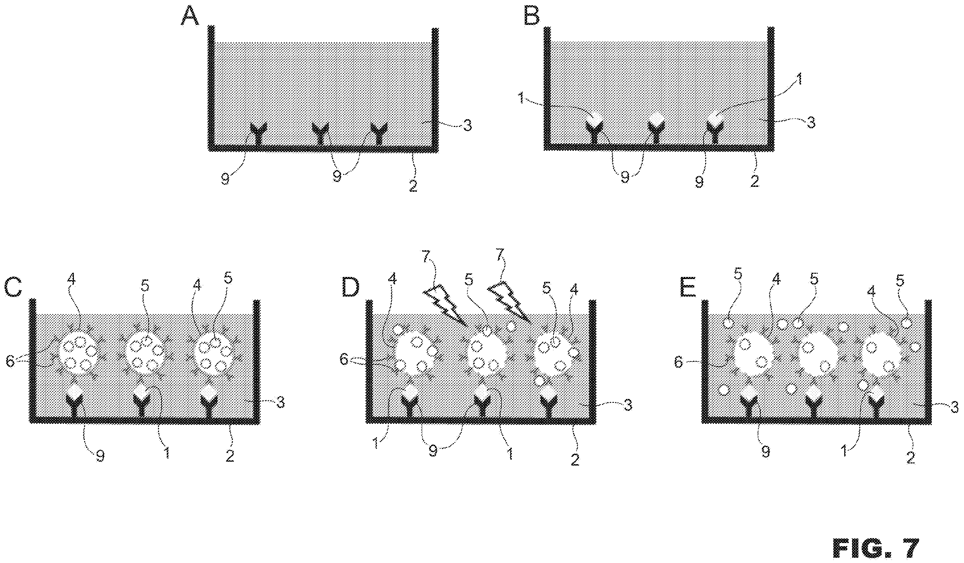

Figure 7 illustrates a further embodiment of the method according to the invention. The details similar or equal to those already described in figure 6 are indicated for the sake of simplicity by the same reference numbers.

In particular, in this case the analyte 1 is now adhered to the solid support 2 by means of a secondary ligand 9 (Fig. 7A-7B).

A suspension of liposomes 4 adequately functionalized with a primary ligand 6 (e.g. secondary antibody) and containing an aqueous solution 5 having a pH different from that of the solution 3 is then added to bind the analyte (Fig. 7C).

At this point, before rupture of the liposomes, a first pH measurement of the solution 3 is taken.

The release of the aqueous solution 5 is induced by an external stimulus 7, in this case for example heat (Figure 7D).

Lastly, after a period of time adequate for complete release of the aqueous solution 5 contained in the liposomes into the solution 3 (Fig. 7E), a second pH measurement is taken which is then compared with the pH before release of the solution 5.

By measuring the pH of the solution after rupture of the liposomes, the quantity of liposomes bound to the ligand is easily quantifiable using the calibrations illustrated above.

Further characteristics of the present invention will become clear from the following description of some purely illustrative and non-limiting examples.

EXAMPLE 1

PREPARATION OF THE LIPOSOMES

The liposomes of the present invention are obtained by means of the thin film hydration method where the phospholipidic, polymeric or dendrimeric mixture is dissolved in organic solvent subsequently removed under a vacuum by means of a rotavapor. The hydration solution containing the material to be encapsulated in the liposomes is added to the film, and multilamellar liposomes are formed by the action of a vortex.

The sonication or extrusion of the multilamellar liposomes leads to the formation of SUV (diameter 40-100 nm) or LUV (diameter 100-500 nm) according to the conditions.

The liposomes can be formulated so as to include almost all the phospholipids available on the market. A typical lipidic mixture used in formation of the liposomes of the invention is the following: 95% 1,2-dipalmitoyl-sn-glycero-3-phosphocholine (DPPC), 5% 1,2 -distearoyl-sn-glycero-3-phosphoethanolamine-N-[methoxy(polyethyleneglycol)-2000] (DSPE-PEG2000-Methoxy).

The lipids that form the liposome are dissolved in an inert organic solvent or in a mixture of solvents to be easily dissolved.

The aqueous solution used in the formation of the liposomes of the present invention contains a basic or acid compound or a buffer solution in a concentration ranging from 0.001 to 1.00 M.

After formation of the liposomes, the suspension is neutralized by adding an equivalent of $H_3O^+$ or $OH^-$ if the hydration solution contains a base or a strong acid respectively, without modifying the osmolarity of the solution. Otherwise, if the internalized compound is a buffer or a weak base or a weak acid, the purification from non-trapped molecules can occur by means of: i) dialysis against iso-osmolar NaCl solution, ii) ultrafiltration and suspension in an iso-osmolar NaCl solution.

Another type of liposomes that can be used in the present invention are the giant unilamellar vesicles (GUVs), the preparation method of which is described in the literature.[17]

The liposomes of the invention are also prepared with a membrane containing ligand molecules able to bind specifically and with high affinity to the analytes.

Many methods are available for attaching ligand molecules on the surface of the lipidic or polymeric vesicles.[18,19]

EXAMPLE 1A:

The liposomal suspension of example 1A is characterized by a phospholipidic membrane consisting of DPPC/DSPE-PEG2000-Methoxy (molar ratio 95/5) and traps inside it a solution of NaOH 1 mM. The preparation was carried out by dissolving 16.64 mg of DPPC and 3.36 mg of DSPE-PEG2000-Methoxy (purchased from AvantiPolar Lipids) in 2 ml of chloroform. The lipidic mixture was dried under a vacuum in a rotavapor system and the thin film formed on the wall of the round-bottom flask was hydrated with a 1 mM solution of NaOH. The solution was agitated on vortex to form multilamellar vesicles and then ultrasound-treated to form large unilamellar vesicles (LUV). The non-trapped excess NaOH was neutralized by adding an HCl equivalent. Three samples at different concentrations were heated to 55°C for 10 minutes to allow the content to flow out of the internal compartment.

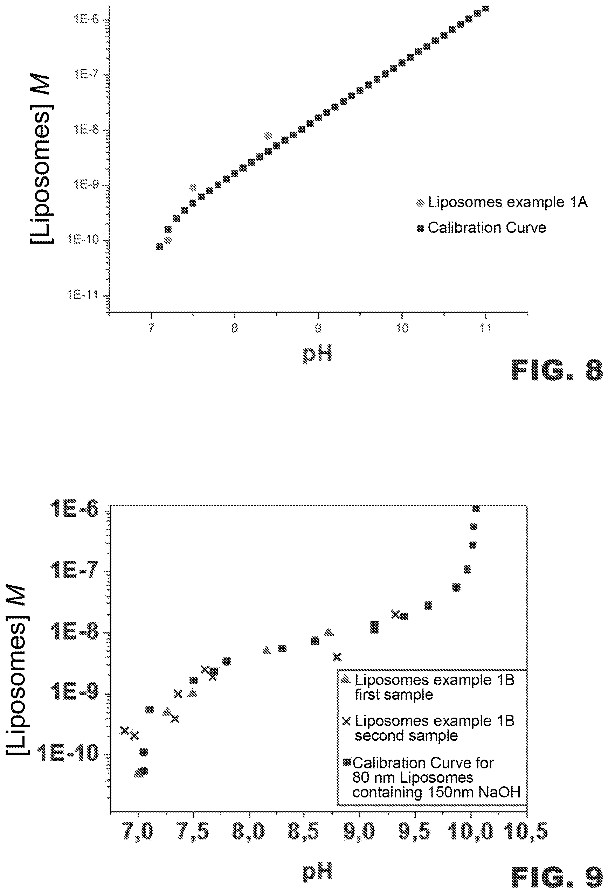

The pH was subsequently measured and the results are reported in Figure 8 where the pH values of the solution of example 1A are shown as circles and the squares represent the calibration curve of Figure 1.

EXAMPLE 1B:

The liposomal suspension of example 1B is characterized by a phospholipidic membrane consisting of DPPC/DSPE-PEG2000-Methoxy (molar ratio 95/5) and traps inside it a solution of NaHCOs 150 mM at pH 10.0. The preparation was carried out by dissolving 16.64 mg of DPPC and 3.36 mg of DSPE-PEG2000-Methoxy (purchased from AvantiPolar Lipids) in 2 ml of chloroform. The lipidic mixture was dried under a vacuum in a rotavapor system and the thin film formed on the wall of the round-bottom flask was hydrated with a solution of NaHCOs 150 mM at pH 10.0. The solution was agitated on vortex to form multilamellar vesicles and then ultrasound treated to form large unilamellar vesicles (LUV). The liposome suspension was dialysed against a solution of NaCl 150 mM at pH 7.0. The concentration was calculated and several samples were prepared at different dilution, adjusting the pH to 7.0 if necessary.

Each sample was heated to 55°C for 10 minutes to allow the content to flow out of the inner compartment.

Subsequently, the pH was measured and the results are reported in Figure 9 (triangles).

All the procedures were repeated on a second preparation of the same liposomes and the results are shown in Figure 9 (crosses). The overlapping of the experimental curve with the one extrapolated from the calibration relative to NaHCOs (Figure 9 squares) for a liposome having the same dimension and bicarbonate load shows good coherence between the two data groups.

EXAMPLE 1C:

In the case of Example 1C giant liposomes were prepared according to the gentle hydration method.

In short: i) DPPC and DSPE-PEG2000-Methoxy (molar ratio 97/3) were dissolved in chloroform, ii) the mixture of phospholipids is placed on the bottom of a flask and dried using a nitrogen flow, iii) the hydration solution (NaHCOs 0.2 M) is gently poured into the flask, iii) the lipids are hydrated, in the absence of mechanical stress, at 60°C for 2 hours, iv) once recovered, the liposomes are centrifuged at 6500 rpm for 15 minutes in order to retain the giant liposomes as pellets and discharge any small liposomes or impurities present with the supernatant.

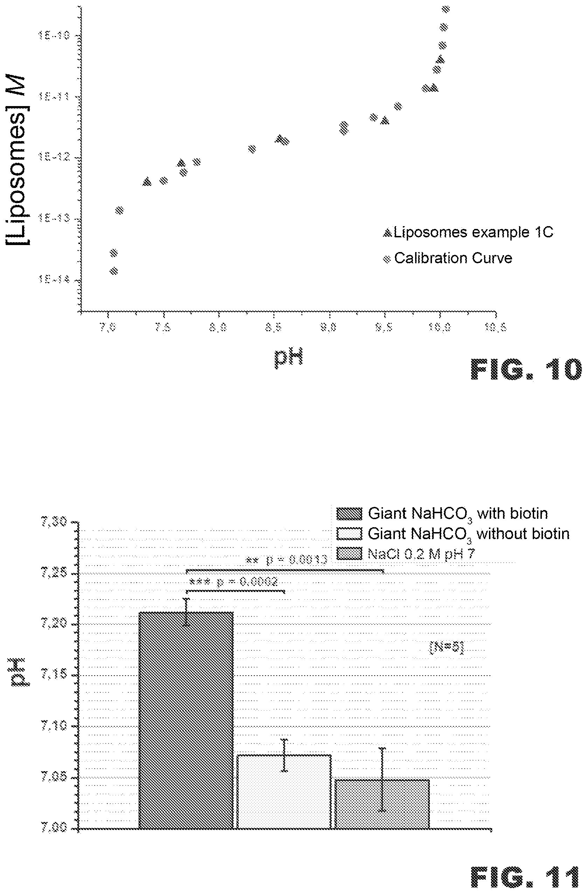

The last operation is repeated several times and the liposomes are re-suspended in 1 ml of NaCl 0.2 M at pH 7.0. The suspension of giant liposomes was then diluted several times and TRITON-X at pH 7.0 was added to each solution to induce the release of their content. The pH of the diluted solutions was measured before and after the addition of TRITON-X (Figure 10 triangles) and the results accord excellently with the calibration reported in Figure 4 (circles).

EXAMPLE 2

EVALUATION OF STABILITY OF THE VESICLES

The liposomes of examples 1B and 1C were tested for their stability to release of content in solutions of NaCl 0.2 M. The liposomes prepared recently, after being purified, underwent continuous pH measurements for three hours. Both the formulations (Examples 1B and 1C) showed no significant increase in the pH value, demonstrating that during this period no OH⁻ were released from the inner liposomal compartment. All these samples at the end of the pH monitoring (pH 7.0) were heated to 55°C for ten minutes and the pH was then measured. A portion of the master solution of freshly prepared liposomes was also heated to 55°C for ten minutes as a reference and the final pH was measured. The procedure was repeated three times on each sample: from the statistical analysis, it can be said that the final pH is the same for all and the same as the master solution, thus showing that the liposomal content was maintained inside the vesicles. The stability of the liposomes of Example 1C was also tested on human serum. The liposomes were incubated with human serum for 1 hour and then washed extensively with NaCl 0.2 M at pH 7.0. Subsequently they were heated to 55°C for ten minutes and the pH was measured. As a control, the same experimental condition was applied to a suspension of liposomes of Example 1C replacing the human serum with NaCl 0.2 M at physiological pH. The final value of the pH was statistically the same in both cases.

EXAMPLE 3

ANALYTE/LIGAND DIAGNOSTIC TESTS

This paragraph reports an example of a general type of immunoassay using the liposomes of the present invention.

EXAMPLE 3A:

The experiment reported here is an example of analyte/ligand direct dosage (Figure 5). The analyte to be detected is streptavidin which binds directly to the molecules of biotin (ligand) present on the liposomes, to form analyte/ligand complexes. Streptavidin is already adsorbed on the surface of a plate. For this experiment, the microplates coated in streptavidin were purchased from Greiner Bio-One International. The giant liposomes containing a biotinylated phospholipid were prepared according to Example 1C substituting the DSPE-PEG2000-Methoxy with 1,2-distearoyl-sn-glycero-3-phosphoethanolamine-N-[biotinyl(polyethyleneglycol)-2000] (DSPE-PEG2000-Biotin). The liposomes prepared on the spot, at a concentration of approximately $1 \cdot 10^{-11}$ M, were incubated in the microplates for 15 minutes under gentle agitation. The microplates were then washed 5 times with NaCl 0.2 M at pH 7.0. The pH was measured with a microelectrode (METTLER TOLEDO) and was 7.0. At this point a solution containing TRITON-X at pH 7.0 was added to the microplates, the pH was measured 15 minutes later and an increase of 0.2 pH units was detected. In accordance with the calibration reported in Figure 10, this value indicates that approximately $1 \cdot 10^{-13}$ M of liposomes were bound to the plates. These measurements were repeated five times and the results are reported in Figure 11. As a control, the same experiment was repeated using giant liposomes not functionalized on the outer surface with biotin (example 1C) but maintaining the same dimensions and the same content. In this case the pH variation is approximately 0.05 units. As a further control, the microplates were treated only with the washing solution (NaCl 0.2 M pH 7.0). Both the controls were repeated 5 times. The statistical analysis of the results shows that the difference between the functionalized liposomes and control is statistically significant (Student T test, $\rho$ = 0.0002).

REFERENCES

(1) Schuurs, A.; Van, W. B. Process for the Demonstration and Determination of Low Molecular Compounds and of Proteins Capable of Binding These Compounds Specifically. US3850752A, November 26, 1974.

(2) Schuurs, A. H. W.; V7eemen, B. K. V. Method for the Determination of Antigens and Antibodies. US3654090A, April 4, 1972.

(3) Zhang, S.; Garcia-D'Angeli, A.; Brennan, J. P.; Huo, Q. Predicting Detection Limits of Enzyme-Linked Immunosorbent Assay (ELISA) and Bioanalytical Techniques in General. Analyst 2014, 139 (2), 439-445. https://doi.org/10.1039/c3an01835k.

(4) Davies, G. E.; Janata, J. Magnetic Particles for Immunoassay. US4177253A, December 4, 1979.

(5) Updike, S.; Goodfriend, T. Reagent for Radioimmunoassay. US3793445A, February 19, 1974.

(6) Kung, V. T.; Canova-Davis, E. Liposome Immunoassay Reagent and Method. US4622294A, November 11, 1986.

(7) Ren, R.; Cai, G.; Yu, Z.; Tang, D. Glucose-Loaded Liposomes for Amplified Colorimetric Immunoassay of Streptomycin Based on Enzyme-Induced Iron(II) Chelation Reaction with Phenanthroline. Sensors and Actuators B: Chemical 2018, 265, 174-181. https://doi.org/10.1016/j.snb.2018.03.049.

(8) Horie, M.; Yanagisawa, H.; Sugawara, M. Fluorometric Immunoassay Based on PH-Sensitive Dye-Encapsulating Liposomes and Gramicidin Channels. Analytical Biochemistry 2007, 369 (2), 192-201. https://doi.org/10.1016/j.ab.2007.07.007.

(9) Haga, M.; Hoshino, S.; Okada, H.; Hazemoto, N.; Kato, Y.; Suzuki, Y. An Improved Chemiluminescence-Based Liposome Immunoassay Involving Apoenzyme. Chem. Pharm. Bull. 1990, 38 (1), 252-254.

(10) Liang, J.; Wang, J.; Zhang, L.; Wang, S.; Yao, C.; Zhang, Z. Glucose Oxidase-Loaded Liposomes for in Situ Amplified Signal of Electrochemical Immunoassay on a Handheld PH Meter. New J. Chem. 2019, 43 (3), 1372-1379. https://doi.org/10.1039/C8NJ05637D.

(11) Ishikawa, E.; Imagawa, M.; Hashida, S.; Yoshitake, S.; Hamaguchi, Y.; Ueno, T. Enzyme-Labeling of Antibodies and Their Fragments for Enzyme Immunoassay and Immunohistochemical Staining. J Immunoassay 1983, 4 (3), 209-327.

(12) Poznansky, M. J.; Juliano, R. L. Biological Approaches to the Controlled Delivery of Drugs: A Critical Review. Pharmacol. Rev. 1984, 36 (4), 277-336.

(13) Toonen, P. A. H. M.; Crommelin, D. J. A. Immunoglobulins as Targeting Agents for Liposome Encapsulated Drugs. Pharmaceutisch Weekblad Scientific Edition 1983, 5 (6), 269-280. https://doi.org/10.1007/BF02074854 .

(14) Connor, J.; Sullivan, S.; Huang, L. Monoclonal Antibody and Liposomes. Pharmacology & Therapeutics 1985, 28 (3), 341-365. https://doi.org/10.1016/0163-7258(85)90058-0.

(15) Hansen, C. B.; Kao, G. Y.; Moase, E. H.; Zalipsky, S.; Allen, T. M. Attachment of Antibodies to Sterically Stabilized Liposomes: Evaluation, Comparison and Optimization of Coupling Procedures. Biochimica et Biophysica Acta (BBA) - Biomembranes 1995, 1239 (2), 133-144. https://doi.org/10.1016/0005-2736(95)00138-S.

(16) George, A.; Tan, P. Immunoliposomes Formed with Aggregated Antibody. WO2003101427A1, December 11, 2003.

(17) Reeves, J. P.; Dowben, R. M. Formation and Properties of Thin-walled Phospholipid Vesicles. Journal of Cellular Physiology 1969, 73 (1), 49-60. https://doi.org/10.1002/jcp.1040730108.

(18) Zhang, H. Thin-Film Hydration Followed by Extrusion Method for Liposome Preparation. Methods Mol. Biol. 2017, 1522, 17-22. https://doi.org/10.1007/978-1-4939-6591-5_2.

(19) Szoka, F. C. Liposomes with Glycolipid-Linked Antibodies. US4483929A, November 20, 1984.

(20) Liang, Glucose oxidase-loaded liposomes for in situ amplified signal of electrochemical immunoassay on handheld pH meter. New J.Chem., 2019, 43, 1372.

## Claims

1. A method for the dosing of an analyte (1) of interest in a solution (3) comprising the steps of:

   a) providing a solution (3) of said analyte (1) of interest bound to a solid support (2), said solution (3) having a first pH;
   b) placing in contact with said analyte solution (1) a composition of liposomes (4) containing an aqueous solution (5) having a second pH different from the first pH and presenting on the surface at least one ligand (6) specific for said analyte (1) of interest, so as to create a bond between said analyte (1) and said ligand (6);
   c) removing unbound liposomes (4) by washing cycles;
   d) applying an external stimulus (7) so as to cause release of the aqueous solution (5) contained in said liposomes (4) into the solution (3) containing said analyte (1) so as to obtain a third pH;
   e) measuring said third pH;
   f) calculating the dosage of said analyte (1).

2. The method according to the preceding claim, **characterized in that** said analyte (1) and ligand (6) are selected from the group consisting of antigen/antibody, immunoglobulin/protein A, biotin/avidin, hormone/receptor, complementary sequences of nucleic acids, aptamer/target molecule.

3. The method according to any one of the claims 1 or 2, **characterized in that** said bond between said analyte (1) and said ligand (6) is an indirect bond.

4. The method according to claim 3, **characterized in that** said indirect bond occurs by means of a secondary ligand (8).

5. The method according to any one of the claims 1 or 4, **characterized in that** said external stimulus (7) is selected from the group consisting of ultrasounds, heat, presence of surfactants, osmotic stress and freeze-thaw cycles.

## Patentansprüche

1. Verfahren zur Dosierung eines interessierenden Analyten (1)ner Lösung, umfassend die Schritte:

a) Bereitstellen einer Lösung (3) des interessierenden Analyten (1), der an einen festen Träger (2) gebunden ist, wobei die Lösung (3) einen ersten pH-Wert aufweist,

b) In-Kontakt-Bringen einer Zusammensetzung von Liposomen (4) mit der Lösung des Analyten (1), die eine wässrige Lösung (5) enthält, die einen zweiten pH-Wert aufweist, der sich vom ersten pH-Wert unterscheidet, und die auf der Oberfläche mindestens einen für den interessierenden Analyten (1) spezifischen Liganden (6) aufweist, um eine Bindung zwischen dem Analyten (1) und dem Liganden (6) herzustellen;

c) Entfernen von ungebundenen Liposomen (4) durch Waschzyklen;

d) Anlegen eines externen Reizes (7), um die Freisetzung der in den Liposomen (4) enthaltenen wässrigen Lösung (5) in die den Analyten (1) enthaltende Lösung (3) zu bewirken, um einen dritten pH-Wert zu erhalten;

e) Messen des dritten pH-Werts;

f) Berechnen der Dosierung des Analyten (1).

2. Verfahren nach dem vorhergehenden Anspruch, **dadurch gekennzeichnet, dass** der Analyt (1) und der Ligand (6) aus der Gruppe Antigen/Antikörper, Immunglobulin/Protein A, Biotin/Avidin, Hormon/Rezeptor, komplementäre Sequenzen von Nukleinsäuren, Aptamer/Zielmolekül ausgewählt sind.

3. Verfahren nach einem der Ansprüche 1 oder 2, **dadurch gekennzeichnet, dass** die Bindung zwischen dem Analyten (1) und dem Liganden (6) eine indirekte Bindung ist.

4. Verfahren nach Anspruch 3, **dadurch gekennzeichnet, dass** die indirekte Bindung mittels eines sekundären Liganden (8) erfolgt.

5. Verfahren nach einem der Ansprüche 1 oder 4, **dadurch gekennzeichnet, dass** der äußere Stimulus (7) ausgewählt ist aus der Gruppe bestehend aus Ultraschall, Wärme, Anwesenheit von Tensiden, osmotischem Stress und Gefrier-Auftau-Zyklen.

**Revendications**

1. Méthode pour le dosage d'un analyte (1) d'intérêt dans une solution (3) comprenant les étapes consistant à :

a) fournir une solution (3) dudit analyte (1) d'intérêt lié à un support solide (2), ladite solution (3) ayant un premier pH ;

b) placer en contact avec ladite solution d'analyte (1) une composition de liposomes (4) contenant une solution aqueuse (5) ayant un deuxième pH différent du premier pH et présentant sur la surface au moins un ligand (6) spécifique pour ledit analyte (1) d'intérêt, de manière à créer une liaison entre ledit analyte (1) et ledit ligand (6) ;

c) ôter les liposomes (4) non liés par des cycles de lavage ;

d) appliquer un stimulus externe (7) de manière à provoquer la libération de la solution aqueuse (5) contenue dans lesdits liposomes (4) dans la solution (3) contenant ledit analyte (1) de manière à obtenir un troisième pH ;

e) mesurer ledit troisième pH ;

f) calculer le dosage dudit analyte (1).

2. Méthode selon la revendication précédente, **caractérisée en ce que** ledit analyte (1) et le ligand (6) sont sélectionnés dans le groupe consistant en un antigène/un anticorps, une immunoglobuline/une protéine A, une biotine/une avidine, une hormone/un récepteur, des séquences complémentaires d'acides nucléiques, un aptamère/une molécule cible.

3. Méthode selon l'une quelconque des revendications 1 ou 2, **caractérisée en ce que** ladite liaison entre ledit analyte (1) et ledit ligand (6) est une liaison indirecte.

4. Méthode selon la revendication 3, **caractérisée en ce que** ladite liaison indirecte se produit au moyen d'un ligand secondaire (8).

5. Méthode selon l'une quelconque des revendications 1 à 4, **caractérisée en ce que** ledit stimulus externe (7) est sélectionné dans le groupe consistant en des ultrasons, de la chaleur, la présence de tensioactifs, un stress osmotique et des cycles de gel-dégel.

**FIG. 1**

**FIG. 2**

FIG. 3

FIG. 4

FIG. 5

FIG. 6

EP 3 977 124 B1

FIG. 7

FIG. 8

FIG. 9

**FIG. 10**

**FIG. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IT 102019000007222
- US 4962022 A
- US 3850752 A
- US 3654090 A
- US 4177253 A
- US 3793445 A
- US 4622294 A
- WO 2003101427 A1
- US 4483929 A

### Non-patent literature cited in the description

- **HORIE et al.** *Analytical Biochemistry,* 2007, vol. 369, 192-201
- **SCHUURS, A. ; VAN, W. B.** *Process for the Demonstration and Determination of Low Molecular Compounds and of Proteins Capable of Binding These Compounds Specifically*
- **SCHUURS, A. H. W. ; V7EEMEN, B. K. V.** *Method for the Determination of Antigens and Antibodies*
- **ZHANG, S. ; GARCIA-D'ANGELI, A. ; BRENNAN, J. P. ; HUO, Q.** Predicting Detection Limits of Enzyme-Linked Immunosorbent Assay (ELISA) and Bioanalytical Techniques in General. *Analyst,* 2014, vol. 139 (2), 439-445
- **DAVIES, G. E. ; JANATA, J.** *Magnetic Particles for Immunoassay*
- **UPDIKE, S. ; GOODFRIEND, T.** *Reagent for Radioimmunoassay*
- **KUNG, V. T. ; CANOVA-DAVIS, E.** *Liposome Immunoassay Reagent and Method*
- **REN, R. ; CAI, G. ; YU, Z. ; TANG, D.** Glucose-Loaded Liposomes for Amplified Colorimetric Immunoassay of Streptomycin Based on Enzyme-Induced Iron(II) Chelation Reaction with Phenanthroline. *Sensors and Actuators B: Chemical,* 2018, vol. 265, 174-181
- **HORIE, M. ; YANAGISAWA, H. ; SUGAWARA, M.** Fluorometric Immunoassay Based on PH-Sensitive Dye-Encapsulating Liposomes and Gramicidin Channels. *Analytical Biochemistry,* 2007, vol. 369 (2), 192-201
- **HAGA, M. ; HOSHINO, S. ; OKADA, H. ; HAZEMOTO, N. ; KATO, Y. ; SUZUKI, Y.** An Improved Chemiluminescence-Based Liposome Immunoassay Involving Apoenzyme. *Chem. Pharm. Bull.,* 1990, vol. 38 (1), 252-254
- **LIANG, J. ; WANG, J. ; ZHANG, L. ; WANG, S. ; YAO, C. ; ZHANG, Z.** Glucose Oxidase-Loaded Liposomes for in Situ Amplified Signal of Electrochemical Immunoassay on a Handheld PH Meter. *New J. Chem.,* 2019, vol. 43 (3), 1372-1379
- **ISHIKAWA, E. ; IMAGAWA, M. ; HASHIDA, S. ; YOSHITAKE, S. ; HAMAGUCHI, Y. ; UENO, T.** Enzyme-Labeling of Antibodies and Their Fragments for Enzyme Immunoassay and Immunohistochemical Staining. *J Immunoassay,* 1983, vol. 4 (3), 209-327
- **POZNANSKY, M. J. ; JULIANO, R. L.** Biological Approaches to the Controlled Delivery of Drugs: A Critical Review. *Pharmacol,* 1984, vol. 36 (4), 277-336
- Immunoglobulins as Targeting Agents for Liposome Encapsulated Drugs. **TOONEN, P. A. H. M. ; CROMMELIN, D. J. A.** Pharmaceutisch Weekblad. 1983, vol. 5, 269-280
- **CONNOR, J. ; SULLIVAN, S. ; HUANG, L.** Monoclonal Antibody and Liposomes. *Pharmacology & Therapeutics,* 1985, vol. 28 (3), 341-365
- **HANSEN, C. B. ; KAO, G. Y. ; MOASE, E. H. ; ZALIPSKY, S. ; ALLEN, T. M.** Attachment of Antibodies to Sterically Stabilized Liposomes: Evaluation, Comparison and Optimization of Coupling Procedures. *Biochimica et Biophysica Acta (BBA) - Biomembranes,* 1995, vol. 1239 (2), 133-144
- **GEORGE, A. ; TAN, P.** *Immunoliposomes Formed with Aggregated Antibody*
- **REEVES, J. P. ; DOWBEN, R. M.** Formation and Properties of Thin-walled Phospholipid Vesicles. *Journal of Cellular Physiology,* 1969, vol. 73 (1), 49-60
- **ZHANG, H.** Thin-Film Hydration Followed by Extrusion Method for Liposome Preparation. *Methods Mol. Biol.,* 2017, vol. 1522, 17-22
- **SZOKA, F. C.** *Liposomes with Glycolipid-Linked Antibodies*

- **LIANG.** Glucose oxidase-loaded liposomes for in situ amplified signal of electrochemical immunoassay on handheld pH meter. *New J.Chem.,* 2019, vol. 43, 1372